# EUROPEAN PATENT SPECIFICATION

(11) **EP 3 505 512 B1**
(45) Date of publication and mention of the grant of the patent: **09.03.2022**
(21) Application number: 18248109.3
(22) Date of filing: 28.12.2018
(51) Int. Cl.: C07D 213/82, A61P 1/16, A61P 31/00, A61P 3/06, A61P 11/00, A61P 27/06, A61P 7/02, A61P 9/00, A61P 29/00

(54) **QUATERNARY NICOTINAMIDE DERVIATIVES AS PRECURSORS FOR RELEASE OF MNA**
QUATERNÄRE NIKOTINAMIDDERIVATE ALS VORSTUFEN ZUR FREISETZUNG VON MNA
DÉRIVÉS DE NICOTINAMIDE QUATERNAIRES EN TANT QUE PRÉCURSEURS POUR UNE LIBÉRATION DE MNA

(30) Priority: 31.12.2017 PL 42407317
(43) Date of publication of application: 03.07.2019
(73) Proprietor: UNIWERSYTET JAGIELLONSKI, 31-007 Kraków (PL)
(72) Inventor: CHLOPICKI, Stefan, 31-138 Kraków (PL); KUS , Kamil, 30-384 Kraków (PL); KALVINS, Ivars, 5050 Ikskile (LV); ADRIANOV, Victor, 1082 Riga (LV); LOZA, Einars, 1013 Riga (LV)

(56) References cited:
- WO-A2-2005/067927
- US-A1- 2015 252 027
- S. JANA ET AL: "Prodrug Design to Improve Pharmacokinetic and Drug Delivery Properties: Challenges to the Discovery Scientists", CURRENT MEDICINAL CHEMISTRY, vol. 17, no. 32, 1 November 2010 (2010-11-01), pages 3874-3908, XP055079989, ISSN: 0929-8673, DOI: 10.2174/092986710793205426
- Christina Erb ET AL: "Formation of N-methylnicotinamide in the brain from a dihydropyridine-type prodrug", Biochemical Pharmacology, vol. 57, no. 6, 1 March 1999 (1999-03-01), pages 681-684, XP055677695, US ISSN: 0006-2952, DOI: 10.1016/S0006-2952(98)00338-4

## Description

The invention concerns pyridine salts derivatives which are O-substituted halides of 3-oxymethylaminocarbonyl-1-methylpyridine and their pharmaceutically acceptable salts. Disclosed compounds may be used as a medicament due to their ability to release the active substance in the form of N1-methylnicotinamide (MNA). Preferably, these compounds may be used in the treatment or prevention of diseases associated with vascular endothelial dysfunction. The invention also concerns the method of MNA release and the use of pyridine salts derivatives for the release of MNA.

It is well known that MNA is a metabolite of niacin, which is an endogenous modulator of prostacyclin release (Chlopicki S et al. The British Journal of Pharmacology 2007; 152(2):230-39) and shows numerous pharmacological activities dependent on the COX-2/PGI₂ pathway. In particular, MNA has antithrombotic (Chlopicki S et al. 2007, *op. cit*.), anti-inflammatory (Bryniarski K et al. European Journal of Pharmacology 2008; 578(2-3):332-38) and gastroprotective properties (Brzozowski T et al. Pharmacology 2008; 326(1): 105-16), improves exercise endurance (Przyborowski K et al. PLoS ONE 2015; 10(6): 1-15) and its therapeutic effect can be in many cases mechanistically linked to the improvement of vascular endothelial function (Bartuś M et al. Pharmacological Reports 2008; 60(1): 127-38; Bar A et al. Front Pharmacol 2017;8). Thus, the therapeutic effect of MNA may be used in all diseases associated with endothelial dysfunction.

The anti-inflammatory effect of 1-methyl-N-hydroxymethylnicotinamide (MNAF), the precursor of MNA, is also known. In addition, MNAF reduces the generation of free radicals and has an antibacterial effect (Adamiec M et al. Pharmacological Reports 2006; 58(2):246-49; Miedzobrodzki J et al. Acta Poloniae Pharmaceutica 2010; 67(5):487-94).

In the state of the art some prodrugs of MNA are disclosed (e.g. WO 2005/067927 A2; S. Jana et al. Current Bedicinal Chemistry, vol. 17, no. 32, pages 3874-3908; US 2015/252027 A1; C. Erb et al. Biochem Pharmacol. 1999 Mar 15;57(6):681-4), but the group of compounds of the invoetnion shows tunable pharmacocinetic properties compared to those known precursos.

On the other hand, the Polish patent application PL 364348 is known for the use of a group of quaternary pyridine salts for the production of a vasoprotective agent for the treatment and/or prophylaxis of conditions or diseases related to endothelial dysfunction, oxidative stress and failure of the endothelial prostacyclin synthesis (PGI₂). This application also covers the group of pyridine salts intended to be used in dietary supplementation and the use of these for the protection of blood vessels in mammals in conditions or diseases related to vascular endothelial dysfunction, oxidative stress to activate endothelial prostacyclin synthesis (PGI2).

Due to the fact that exogenously administered MNA is characterized by poor bioavailability and unfavourable pharmacokinetics profile - including short period of time during which an increase in the concentration of MNA in blood is observed - associated with rapid elimination and short biological half-life in blood, new compounds that would have a different pharmacokinetic profile than the MNA itself are needed. An important factor limiting the rate and degree of absorption is low lipophilicity of MNA, which is an ionized lowmolecular weight compound. This properties hinder the penetration of biological barriers, and the transport of the compound through membranes, otherwise transported only be appropriate transporters. Hydrophilic properties of the compound also results in the rapid elimination of the compound by the kidneys, which is associated with a short period of the compound staying in the body. Therefore, the aim of the invention was to develop a novel group of compounds which are MNA precursors, with a higher lipophilic character, better bioavailability and/or a better profile of released MNA concentration in plasma compared to the administration of the MNA.

The technical problem solved by the invention is the development of novel compounds MNA prodrugs that by the hydrolysis of these compounds release an active substance in the form of MNA, but they have different characteristics, including in particular a different profile of MNA plasma concentration profile and different bioavailability as compared to the administration of the MNA itself. Thus, these compounds have the same pharmacological effect as MNA, but do not have pharmacokinetic limitations of the MNA.

It was unexpectedly found that chemical structures with the general formula (I) show the activity specified above. Therefore, the subject of the invention are pyridine salts derivatives which are O-substituted halides of 3-oxymethylaminocarbonyl-1-methylpyridine with the formula (I): 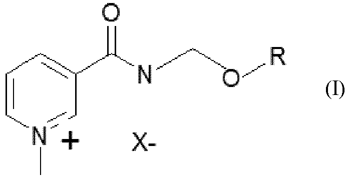 where:
**R** is the substituent selected from the group: C(O)R¹ and -(CH₂)R², where:
   **R1** is the substituent selected from a group including: lower straight-chain or branched C1-C4 alkane, adamantyl, phenyl or trilkoxophenyl trimethoxyphenyl group, lower straight-chain or branched C1-C5 alkane substituted with a COOH or COOR³ group, where:
      **R3** is: lower C1-C4 alkane,
   **R2** is the substituent selected from the group including: H, lower C1-C3 alkane.
**X is:** F-, Cl-, Br-, I- or other pharmaceutically acceptable counterion.

According to the invention, preferred derivatives of pyridine salts selected from the group:
3-(Acetoxymethylcarbamoyl)-1-methylpyridinium iodide;
3-[(2,2-dimethylpropionyl oxymethyl)carbamoyl]-1-methylpyridinium iodide;
3-(benzoyloxymethylcarbamoyl)-methylpyridinium iodide;
3-(isobutanoyloxymethylcarbamoyl)-1-methylpyridinium iodide;
iodide [(1-methylpyridine-1-ium-3 -carbonyl)amino]methyl 3,4,5-trimethoxybenzoate.

The subject of invetion is also iodide [(1-methylpyridine-1-ium-3-carbonyl)amino] methyl-1-methylcyclohexanecarboxylate.

Preferably, derivatives of pyridine salts, according to the invention selected from the group:
4-[[(1-methylpyridin-1-ium-3-carbonyl)amino]methoxy -4-oxobutanoic acid iodide;
3-({[(5-ethoxy-5-oxopentanoyl)oxy]methyl}carbamoyl)-1-methylpyridinium iodide ;
3-({[(4-ethoxy-4-oxobutanoyl)oxy]methyl}carbamoyl)-1-methylpyridinium iodide .

The invention also concerns the method of releasing N1-methylnicotinamide (MNA), which is characterized by the following steps: pyridine salts derivatives, as defined above, are incubated in a shaking water bath at 37°C for 120 minutes, then samples are taken 1, 5, 15, 30, 60, 90 and 120 minutes after the start of incubation, by taking 50 µL of a sample to which 500 µL of acetonitrile cooled to -20°C was added to interrupt the hydrolysis reaction, then samples were cooled for 20 minutes at 4°C for protein precipitation, and then centrifuged under the following conditions: 4°C, 15,000 x rpm, 15 min, and the supernatant was transferred to separate chromatographic tubes and 5 µL was applied to the chromatographic column. Preferably, when MNA release is studied in PBS buffer - testing hydrolytic stability in solution and/or in rat plasma - additionally testing plasma esterase-dependent hydrolysis. The subject of the invention is also the use of pyridine salts derivatives as defined above for the release of MNA.

The invention also concerns pyridine salts derivatives according to the invention in use as a medicament, preferably in use for the treatment or prevention of diseases associated with vascular endothelial dysfunction, defined as an impairment of endothelial nitric oxide (NO) production, which is accompanied by activation of thrombotic and inflammatory processes of the endothelium and blood vessel wall, selected from the group: atherosclerosis in patients with stable coronary artery disease, ischemic stroke episode or limb atherosclerosis, including thromboembolic arteritis; hypercholesterolemia, hypertriglyceridemia; thrombosis other than arterial atherosclerosis, in particular thrombosis associated with implantation of metal vascular prostheses, coronary bypass surgery, haemodialysis, venous thromboembolism; the condition with the current risk factor of atherosclerosis selected from the group: hypercholesterolemia, hypertension, hyperhomocysteinemia, obesity, mental stress, infections, vessel graft atherosclerosis in organ transplantation; chronic heart failure, pulmonary hypertension, nephrotic syndrome, chronic renal failure, adult acute respiratory distress syndrome, cystic fibrosis, chronic obstructive pulmonary disease, pre-eclampsia, erection disorders, Stein-Leventhal syndrome, sleep apnoea, systemic lupus erythematosus, sickle cell anaemia, peptic ulcer disease, glaucoma, chronic liver disease, viral hepatitis, hepatic steatosis, as well as cancer metastasis.

The term "compounds according to invention" used in this description refers to compounds with formula (I).

The term "pharmaceutically acceptable counterion" refers to counter ions forming salts that are - in terms of medical evaluation - suitable for use in contact with human and lower animal tissues without excessive toxicity, irritation or allergic response. Pharmaceutically acceptable salts, e.g. metal salts and acid addition salts, with both organic and inorganic acids, are known in the field of pharmaceuticals. Representative examples of pharmaceutically acceptable acid addition salts include, but are not limited to, hydrochlorides, bromides, sulphates, nitrates, phosphates, sulfonates, methane sulfonates, formates, tartrates, maleates, citrates, benzoates, salicylates, ascorbates, acetates and oxalates.

The invention covers all possible optical isomers, e.g. diastereoisomers and compound enantiomers. Furthermore, the invention covers both individual isomers, as well as any mixtures of them, e.g. racemic mixtures. The individual isomers can be obtained using the appropriate isomeric forms of the starting substance or can be separated after the final compound has been created according to typical separation methods. For the separation of optical isomers, e.g. enantiomers, from their mixtures, typical separation methods may be used., e.g. fractional crystallisation.

In order to make it easier to understand the subject of the invention, some of its preferred embodiments have been described below. They are only illustrative embodiments and should not be equated with the full scope of an invention. The object of the invention is also shown in figures on which:
- fig. 1: shows chemical structures of compounds quickly releasing MNA;
- fig. 2: shows chemical structures of metabolically stable compounds releasing small amounts of MNA;
- fig. 3: shows chemical structures of compounds which, according to the invention, have a structure of betaines;
- fig. 4: is a graph showing dependence of MNAF and MNA concentration on the incubation time in PBS buffer for compounds quickly releasing MNA;
- fig. 5: is a graph showing dependence of MNAF and MNA concentrations on time incubation in fresh rat plasma for compounds quickly releasing MNA;
- fig. 6: is a graph showing dependence of the concentration of the examined compounds on time incubation in PBS and in rat plasma for very stable compounds releasing small amounts of MNA;
- fig. 7: is a graph showing plasma concentration of parent substances in rat plasma for betaine-type MNA- releasing compounds.

### Examples

The general method of obtaining compounds according to the invention is shown in Diagram 1:

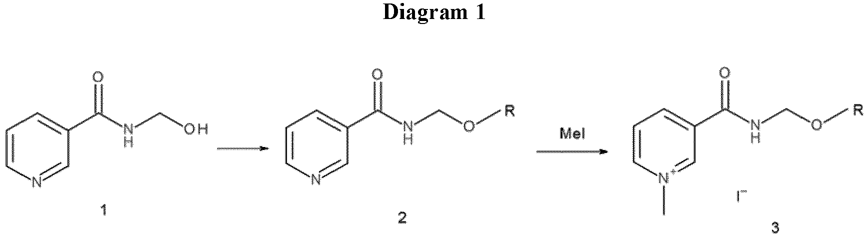

NMR spectra were recorded at ambient temperature using the Varian 400 OXFORD NMR spectrometer (400 MHz). LC-MS was performed using Acquity UPLC system (Waters) combined with Micromass Quatro microTM API (Micromass) tandem mass spectrometer operating in ESI positive ionization mode (ionisation by electrospray) and using Acquity UPLC BEH HILIC column (1.7 µm, 2.1 x 100 mm) on a gradient of 80-50% acetonitrile / 10 mM ammonium acetate (pH 4). Elemental analyses were obtained with the Carlo Erba EA 1108. Melting temperatures were measured in the Gallenkamp or OptiMelt melting temperature apparatus and are uncorrected. Various reagents and solvents were purchased from Sigma-Aldrich, Acros Organic, Alfa Aesar and Apollo Scientific. Silicagel, 0.035-0.070 mm, (Acros) was used for column chromatography.

Table 1 shows methods of synthesis and properties of compounds according to the invention (all labelled with "C-number") and further reference compounds needed for their preparation.

**Table 1**

| *Symbol* | *Method of synthesis and properties* |
|---|---|
| | N-(hydroxymethyl) pyridine-3-carboxamide (**S1**) was obtained as described in: N. Singh, V. K. Pandey, R. Gupta. Pharma Chemica, 2015, 7 (6), 305-311. |
| | Pyridine-3-carbonylamino methyl acetate (**S2a**) was obtained by the method described in: Ivan A. Natchev Tetrahedron, 1988, 44 (20), 6465-70. |
| | (Pyridine-3-carbonylamino)methyl 2,2-dimethylpropanoate (**S2b**). To a suspension of N-(hydroxymethyl) pyridine-3-carboxamide (**S1**) (1,52 g, 10 mmol) in acetonitrile (20 ml) triethylamine (1,52 g, 15 mmol) was added and then a solution of 2,2-dimethylpropanoyl chloride (1,57 g, 1,3 mmol) in acetonitrile (10 ml) was added during 10 minutes at 15-20°C. The reaction mixture was stirred at room temperature in an argon atmosphere for 24 hours, the precipitate was filtered and washed with acetonitrile (10 ml). The filtrate was evaporated, water (20 ml) was added to the residue and the mixture was extracted with ethyl acetate (3 x 30 ml), washed with saturated NaCl (20 ml), dried (Na₂SO₄) and evaporated. The residue was purified by column chromatography on silica gel using chloroform/methanol as an eluent (10:1), as a result (**S2b**) methyl (pyridine-3-carbonylamino 2,63-dimethylpropanoate) (1,63 g, 69%) was obtained. |
| | (Pyridine-3-carbonylamino)methyl benzoate (**S2c**) was obtained from N- (hydroxymethyl) pyridine-3-carboxamide (**S1**) and benzoyl chloride by a similar protocol to **S2b**, yield 42%. |
| | (Pyridine-3-carbonylamino) methyl 2-methylpropionate (**S2d**) was obtained from N-(hydroxymethyl) pyridine-3-carboxamide (**S1**) and 2-methylpropanoyl chloride by a similar protocol to **S2b**, yield 53%. |
| C-2912 | (Pyridine-3-carbonylamino)methyl 3,4,5-trimethoxybenzoate (**S2e**) was obtained from N-(hydroxymethyl) pyridine-3-carboxamide (**S1**) and 3,4,5-trimethoxybenzoyl chloride by a similar protocol to **S2b**, yield 39%. C₁₇H₁₈N₂O₆ (M: 346.(33); melting point: 134-136°C; ¹H-NMR spectrum 400 MHz: (DMSO-d₆, HMDSO) δ: 3.74 (s, 3H); 3.83 (s, 6H); 5.61 (d, J=6.7 Hz, 2H); 7.23 (s, 2H); 7.55 (ddd, J=0.8 Hz, 4.8 Hz, 8.0 Hz, 1H); 8.27 (ddd, J=1.8 Hz, 2.3 Hz, 8.0 Hz, 1H); 8.75 (dd, J=1.7 Hz, 4.8 Hz, 1H); 9.09 (dd, J=0.8 Hz, 2.3 Hz, 1H); 9.86 (t, J=6.7 Hz, 1H) |
| | (Pyridine-3-carbonylamino)methyl 1-methylcyclohexanecarboxylate (**S2f**) was obtained from N-(hydroxymethyl) pyridine-3-carboxamide(**S1**) and 1-methylcyclohexanecarbonyl chloride by a similar protocol to **S2b**, yield 54%. |
| | (Pyridine-3-carbonylamino)methyl adamantane-1-carboxylate (**S2g**) was obtained from N-(hydroxymethyl) pyridine-3-carboxamide(**S1**) and adamanthane-1-carbonyl chloride by a similar protocol to **S2b**, yield 37%. |
| | N- (Methoxymethyl) pyridine-3-carboxamide (**S2h**) was obtained by the method described in: J. Pernak, B. Mrowczynski, J. Weglewski. Synthesis 1994, (12), 1415-17. |
| | 4-oxo-4-[(pyridin-3-carbonylamino) methoxy] butanoic acid (**S2i**). A mixture of N-(hydroxymethyl) pyridine-3-carboxamide (**S1**) (1.52 g, 10 mmol) and succinic anhydride (1.0 g, 10 mmol) was heated at 125-140°C for 20 min. The reaction mixture was cooled and the product was purified by column chromatography on silica gel using chloroform/methanol as eluent (3: 1), resulting in 4-oxo-4-[(pyridine-3-carbonylamino)methoxy] butanoic acid (**S2i**) ( 1,03 g, 41%). |
| | O1-ethyl O5-[(pyridine-3-carbonylamino)methyl] pentanedioate (**S2j**) was obtained from N-(hydroxymethyl)pyridine-3-carboxamide (**S1**) and ethyl 5-chloro-5-oxo-pentanoate by a similar protocol to **S2b**, yield 49%. |
| | O1-Etyl 04 - [(pyridine-3-carbonylamino)methyl] butanedioate (**S2k**) was obtained from N-(hydroxymethyl) pyridine-3-carboxamide (**S1**) and ethyl 4-chloro-4-oxo-butanoate by a similar protocol to **S2b**, yield 52%. |
| C-2578 | 3-(Acetoxymethylcarbamoyl)-1-methylpyridinium iodide (**S3a**). (Pyridine-3-carbonylamino)methyl acetate (**S2a**) (0,97 g, 5 mmol) was dissolved in acetone (10 ml) and methyl iodide (1,42 g, 10 mmol) was added. The reaction mixture was stirred at room temperature in argon atmosphere for 24 hours, the product was filtered and washed with acetone (2 ml). Yield 1.21 g (72%). C₁₀H₁₃IN₂O₃(M: 336.(13); melting point: 93-95°C; ¹H-NMR spectrum 400 MHz: (DMSO-d₆, HMDSO) δ: 2.04 (s, 3H); 4.40 (s, 3H); 5.35 (d, J=6.6 Hz, 2H); 8.26 (dd, J=6.1 Hz, 8.1 Hz, 1H); 8.92 (dt, J=1.5 Hz, 8.1 Hz, 1H); 9.13 (dt, J=1.5 Hz, 6.1 Hz, 1H); 9.44 (br s, 1H); 10.08 (t, J=6.6 Hz, 1H). |
| C-2647 | 3-[(2,2-dimethylpropionyl oxymethyl)carbamoyl]-1-methylpyridinium iodide (**S3b**) was obtained from (pyridine-3-carbonylamino)methyl 2,2-dimethylpropanoate (**S2b**) by a similar protocol to **S3a**. The product was obtained as oil after evaporation of the reaction mixture. Yield 79% C₁₃H₁₉IN₂O₃ (M: 378.21); 1H-NMR spectrum 400 MHz: (DMSO-d₆, HMDSO) δ: 1.14 (s, 9H); 4.40 (s, 3H); 5.38 (d, J=6.6 Hz, 2H); 8.25 (dd, J=6.1 Hz, 8.1 Hz, 1H); 8.92 (d, J=8.2 Hz, 1H); 9.12 (d, J=6.1 Hz, 1H); 9.4 5 (br s, 1H); 1O.O 9 (t, J=6. 6 H z, 1H) |
| C-2648 | 3-(benzoyloxymethylcarbamoyl)-1-methylpyridinium iodide (**S3c**) was obtained from (pyridine-3-carbonylamino)methyl benzoate (**S2c**) by a similar protocol to **S3a**. Yield 71%. C₁₅H₁₅IN₂O₃ (M: C15H15IN2O3 (M: 112-114°C; ¹H-NMR spectrum 400 MHz: (DMSO-d₆, HMDSO) δ: 4.40 (s, 3H); 5.65 (d, J=6.6 Hz, 2H); 7.51-7.59 (m, 2H); 7.65-7.72 (m, 1H); 7.93 -8.00 (m, 2H); 8.26 (dd, J=6.1 Hz, 8.1 Hz, 1H); 8.95 (d, J =8.2 Hz, 1H); 9.12 (d, J=6.1 Hz, 1H); 9.46 (br s, 1H); 10.25 (t, J=6.6 Hz, 1H) |
| C-2649 | 3-(isobutanoyloxymethylcarbamoyl)-1-methylpyridinium iodide (**S3d**) was obtained from (pyridine-3-carbonylamino)methyl 2-methylpropanoate (**S2d**) by a similar protocol to **S3b.** Yield 77%. C₁₂H₁₇IN₂O₃ (M: 364.18); ¹H-NMR spectrum 400 MHz: (DMSO-d₆, HMDSO) δ: 1.90 (d, J=7.0 Hz, 6H); 2.54 (septet, J=7.0 Hz, 1H); 4.40 (s, 3H); 5.38 (d, J=6.6 Hz, 2H); 8.26 (dd, J=6.1 Hz, 8.1 Hz, 1H); 8.92 (d, J=8.2 Hz, 1H); 9.13 (d, J=6.1 Hz, 1H); 9.44 (br s, 1H); 10.09 (t, J=6.6 Hz, 1H) |
| C-2942 | 3-({[(3,4,5-trimethoxybenzoyl)oxy]methyl}carbamoyl)-1 methylpyridinium iodide (**S3e**) was obtained from (pyridine-3-carbonylamino)methyl 3,4,5-trimethoxybenzoate (**S2e**) by a similar protocol to **S3a**. Yield 55%. C₁₈H₂₁IN₂O₆ (M: 488.27); melting point: 142-144°C; ¹H-NMR spectrum 400 MHz: (CDCl₃, HMDSO) δ: 3.88 (s, 3H); 3.88 (s, 6H); 4.63 (s, 3H); 5.73 (d, J=6.7 Hz, 2H); 7.27 (s, 2H); 8.10 (dd, J=6.2 Hz, 8.2 Hz, 1H); 8.89 (d, J=6.2 Hz, 1H); 9.05 (d, J=8.2 Hz, 1H); 9.59 (t, J=6.7 Hz, 1H); 10.26 (s, 1H) |
| C-2959 | iodide [(1-methylpyridine-1-ium-3-carbonyl)amino] methyl-1-methylcyclohexanecarboxylate (**S3f**) was obtained from (pyridine-3-carbonylamino) methyl-1-methylcyclohexanecarboxylate (**S2f**) by a similar protocol to **S3b**. Yield 74%. C₁₆H₂₃IN₂O₃ (M: 418.27); melting point: oil; ¹H-NMR spectrum 400 MHz: (CDCl₃, HMDSO) δ: 1.41 (s, 3H); 1.17-1.40 (m, 5H); 1.40-1.56 (m, 3H); 1.91-2.01 (m, 2H); 4.69 (s, 3H); 5.47 (d, J=6.5 Hz, 2H); 8.21 (dd, J=5.6 Hz, 7.9 Hz, 1H); 9.10 (d, J=7.9 Hz, 1H); 9.26 (d, J=5.6 Hz, 1H); 9.35 (t, J=6.5 Hz, 1H); 10.04 (s, 1H) |
| C-2808 | 3-[(adamanthan-1-carbonyl oxymethyl)carbamoyl]-1-methylpyridinium iodide (**S3g**) was obtained from (pyridin-3-carbonylamino) methyl adamanthanthan-1-carboxylate (**S2g**) by a similar protocol to **S3b**. Yield 81%. C₁₉H₂₅IN₂O₃ ^{•}0.5 H₂O (M: 465.33); ¹H-NMR spectrum 400 MHz: (DMSO-d₆, HMDSO) δ: 1.60-1.72 (m, 6H); 1.76-1.83 (m, 6H); 1.92-2.00 (m, 3H); 4.40 (s, 3H); 5.36 (d, J=6.5 Hz, 2H); 8.25 (dd, J=6.1 Hz, 8.1 Hz, 1H); 8.92 (dt, J=1.4 Hz, 8.1 Hz, 1H); 9.12 (d, J=6.1 Hz, 1H); 9.45 (br s, 1H); 10.08 (t, J=6.5 Hz, 1H) |
| C-2823 | 3-(methoxymethylcarbamoyl)-1-methylpyridinium iodide (**S3h**) was obtained from N-(methoxymethyl) pyridine-3-carboxamide (**S2h**) by a similar protocol to **S3a**. Yield 71%. C₉H₁₃IN₂O₂ (M: 308.(12); melting point: 101-103°C; ¹H-NMR spectrum 400 MHz: (DMSO-d₆, HMDSO) δ: 3.29 (s, 3H); 4.41 (s, 3H); 4.75 (d, J=6.2 Hz, 2H); 8.27 (dd, J=6.1 Hz, 8.2 Hz, 1H); 8.92 (dt, J=1.6 Hz, 8.2 Hz, 1H); 9.12 (d, J=6.1 Hz, 1H); 9.43 (br s, 1H); 9.80 (t, J=6.2 Hz, 1H) |
| C-3008 | 4-[[(1-methylpyridin-1-ium-3-carbonyl)amino] methoxy]-4-oxobutanoic acid iodide (**S3i**) was obtained from 4-oxo-4-[(pyridine-3-carbonylamino)methoxy]butane (**S2i**) by a similar protocol to **S3b**. Yield 74%. C₁₂H₁₅IN₂O₅ (M: 394.16), ₁H-NMR spectrum 400 MHz: (DMSO-d₆, HMDSO) δ: 2.46-2.58 (m, overlapped with DMSO, 4H); 4.40 (s, 3H); 5.38 (d,1=6.6 Hz, 2H); 8.25 (dd,1=6.1 Hz, 8.1 Hz, 1H); 8.91 (d, 8.1 Hz, 1H);9.12(d,1=6.1 Hz, 1H);9.43 (s, 1H); 10.10 (t, J=6.6 Hz, 1H); 12.22 (br s, 1H); (CD₃OD, HMDSO) δ: 2.65 (s, 4H); 4.51 (s, 3H); 5.51 (s, 2H); 8.24 (dd, 1=6.2 Hz, 8.0 Hz, 1H); 8.96 (d, 8.0 Hz, 1H); 9.09 (d, 1=6.2 Hz, 1H); 9.41 (s, 1H) |
| C-3027 | 3-({[(5-ethoxy-5-oxopentanoyl)oxy]methyl}carbamoyl)-1 methylpyridinium iodide (**S3j**) was obtained from ethyl-[N-methyl-(1-methylnicotinic amide)] pentanedioc acid diester iodide (**S2j**) by a similar protocol to **S3b**. Yield 78%. C₁₅H₂₁IN₂O₅ (M: 436.25); ¹H-NMR spectrum 400 MHz: (CDCl₃, HMDSO) δ: 1.23 (t, J=7.2 Hz, 3H); 1.86 (quintet, J=7.4 Hz, 2H); 2.33 (t, J=7.4 Hz, 2H); 2.38 (t, J=7.4 Hz, 2H); 4.09 (q, J=7.2 Hz, 2H); 4.67 (s, 3H); 5.44 (d, J=6.7 Hz, 2H); 8.26 (dd, J=6.1 Hz, 8.1 Hz, 1H); 9.09 (dt, J=1.5 Hz, 8.2 Hz, 1H); 9.30 (dt, J=1.2 Hz, 6.1 Hz, 1H); 9.37 (t, J=6.7 Hz, 1H); 9.91 (s, 1H); ¹³C-NMR spectrum 400 MHz: (CDCl₃, HMDSO) δ: 14.2, 19.9, 33.1, 33.2, 49.8, 60.5, 64.1, 128.3, 133.1, 145.0, 145.2, 147.6, 162.2, 172.6, 173.0 |
| C-3029 | 3-({[(4-ethoxy-4-oxobutanoyl)oxy]methyl}carbamoyl)-1 methylpyridinium iodide (**S3k**) was obtained from Ethyl-[N-methyl-(1-methylnicotinic amide)] butanedioc acid diester iodide (**S2k**) by a similar protocol to **S3b**. Yield 74%. C₁₄H₁₉IN₂O₅ (M: 422.22); ¹H-NMR spectrum 400 MHz: (CDCl₃, HMDSO) δ: 1.24 (t, J=7.2 Hz, 3H); 2.58-2.67 (m, 4H); 4.11 (q, J=7.2 Hz, 2H); 4.66 (s, 3H); 5.46 (d, J=6.7 Hz, 2H); 8.21 (dd, J=6.1 Hz, 8.1 Hz, 1H); 9.08 (dt, J=1.5 Hz, 8.2 Hz, 1H); 9.19 (dt, J=1.2 Hz, 6.1 Hz, 1H); 9.36 (t, J=6.7 Hz, 1H); 9.99 (s, 1H) |

The compounds from the group of pyridine salts derivatives, according to the invention, have in their structure an ester group situated in such a way that as a result of hydrolysis of the parent compound they released the molecule of a stable metabolite - MNAF, which is then transformed into MNA in a subsequent hydrolysis reaction catabolised by esterase, as shown in Diagram 2 below.

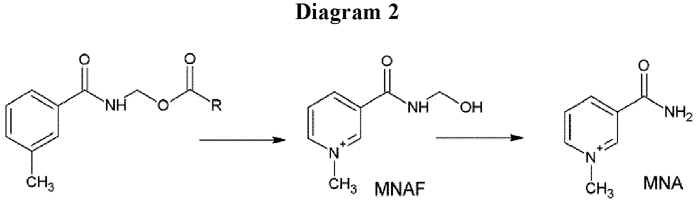

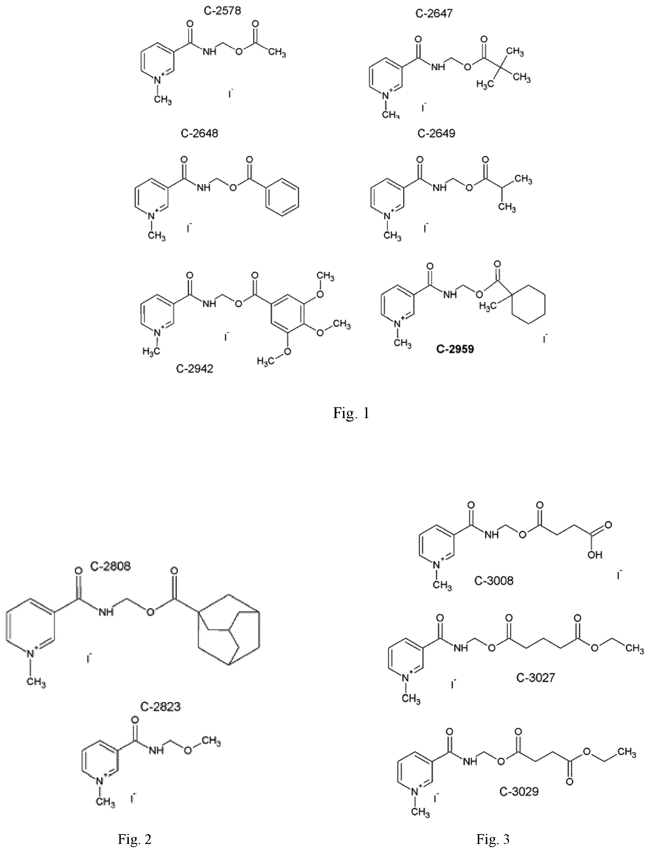

The type of inserted R substituent attached to the leading structure defined by formula I affects the stability of the parent compound and the kinetics of the release of the active compound. Depending on the structure, the compounds which are the subject of the invention show additional properties, such as (a) quick release of an intermediate compound MNAF, which is then transformed into MNA (a group of compounds illustrated in fig. 1 including structures C-2578, C-2647, C-2648, C-2649, C-2942, C-2959); (b) metabolic stability - they hydrolyse very slowly (a group of compounds illustrated in fig. 2 including structures C-2808, C-2823); (c) with increased lipophilic properties - compounds with betaine structure, i.e. intramolecular organic salts, in which there are simultaneously balancing positive and negative charges (a group of compounds illustrated in fig. 3 including structures C-3008, C-3027, C-3029).

The release of MNA by the examined compounds has been studied in *in vitro* hydrolytic stability tests. For that purpose, the studied compounds were incubated: (1) in PBS buffer - testing hydrolytic stability in solution; (2) in rat plasma - testing additionally esterase dependent hydrolysis. Incubation was carried out in a shaking water bath at 37°C. The studied compounds were added to the prepared biological matrix and incubated for 120 min. Samples were collected 1, 5, 15, 30, 60, 90 and 120 min. after the beginning of incubation, by taking 50 µL of a sample to which 500 µL of acetonitrile cooled to -20°C was added to stop the hydrolysis reaction. The samples were cooled for 20 minutes at 4°C in order to precipitate proteins and then centrifuged under the following conditions: 4°C, 15,000 x rpm, 15 min. The supernatant was transferred to separate chromatographic tubes and 5 µL was injected onto chromatographic column.

The analytical system used for determination of MNA and MNAF concentrations consisted of the UltiMate 3000 liquid chromatograph (Dionex, Thermo Scientific) and the Quantum Ultra TSQ mass spectrometer (Thermo Scientific). For chromatographic separation the Aquasil C18 (4.6 x 150 mm, 5 µm, Thermo Scientific) analytical column was used with isocratic mobile phase, a mixture of 0.1% formic acid in acetonitrile and 5mM ammonium formate (80: 20, v/v)) elution ,at the flow rate of 0,8 mL/min. The detection was performed in the selected reaction monitoring (SRM) mode in positive ionization, monitoring the following ion transitions: m/z 137 → 94 (CE=19V) for MNA, m/z 167 → 137 (CE10) for MNAF, m/z 140 → 97 (CE=19V) for MNA-d3 used as internal standard. The parameters of the ion source were as follows: gas pressure 30 psi (*sheath gas*) and 10 psi (*auxiliary gas*)*,* ion spray voltage 4500V, H-ESI ison sourse vaporizer temperature 300°C, capillary temperature 300°C, pressure in the collision chamber 1.5 mTorr (argon).

### (1) MNA fast-release compounds

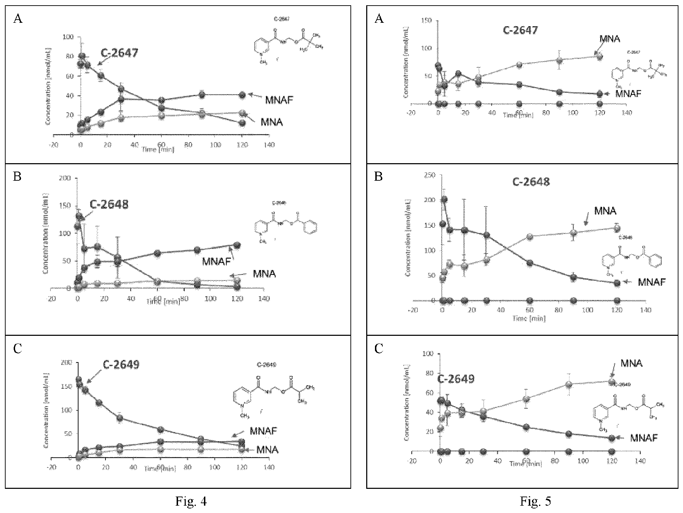

In fig. 1 are shown MNA fast-release compounds which are characterized by very fast hydrolysis to the intermediate metabolite MNAF. During incubation in PBS buffer a gradual decrease in the concentration of parent compounds and simultaneous increase in the concentration of MNAF and MNA metabolites was observed (MNAF and MNA concentration-time curves during incubation of studied compounds are presented in fig. 4). This results prove the chemical hydrolysis of compounds to their metabolites. Chemical hydrolysis of parent compounds to the intermediate metabolite MNAF and MNA was studied by measuring the concentration of the studied compounds incubated for 2 hours in PBS solution at 37°C. Fig. 4 shows an example of concentration-time dependence curves of parent compound - C-2647 (A), C-2648 (B) and C-2649 (C) - blue colour, intermediate metabolite MNAF - red colour and the final active metabolite MNA - green colour.

Incubation of these compounds in fresh rat plasma caused very rapid hydrolysis of parent compounds (the determined concentrations of the parent compounds were below the detection limit already 1 minute after the beginning of incubation) to the intermediate metabolite MNAF (whose concentrations at the beginning of incubation were very high) and then to the MNA (MNAF concentrations decreased with simultaneous increase of MNA concentrations). This indicates a very rapid hydrolysis of the plasma parent compounds to the intermediate MNAF and then to the MNA. Much faster parent compound hydrolysis in plasma than in PBS buffer indicates the contribution of plasma esterases to the decomposition of parent compounds and the conversion of MNAF to MNA. Exemplary concentration-time curves of compounds during incubation in fresh plasma are shown in fig. 5. Hydrolysis of parent compounds to the intermediate metabolite MNAF and MNA dependent on plasma esterases activity was studied by measuring the concentration of the examined compounds incubated for 2 hours in rat plasma at 37°C. In fig. 5 are shown dependence curves of time and concentration of parent compounds - C-2647 (A), C-2648 (B) and C-2649 (C) - blue, intermediate metabolite MNAF - red and the final active metabolite MNA - green.

### (2) Very stable compounds releasing small amounts of MNA

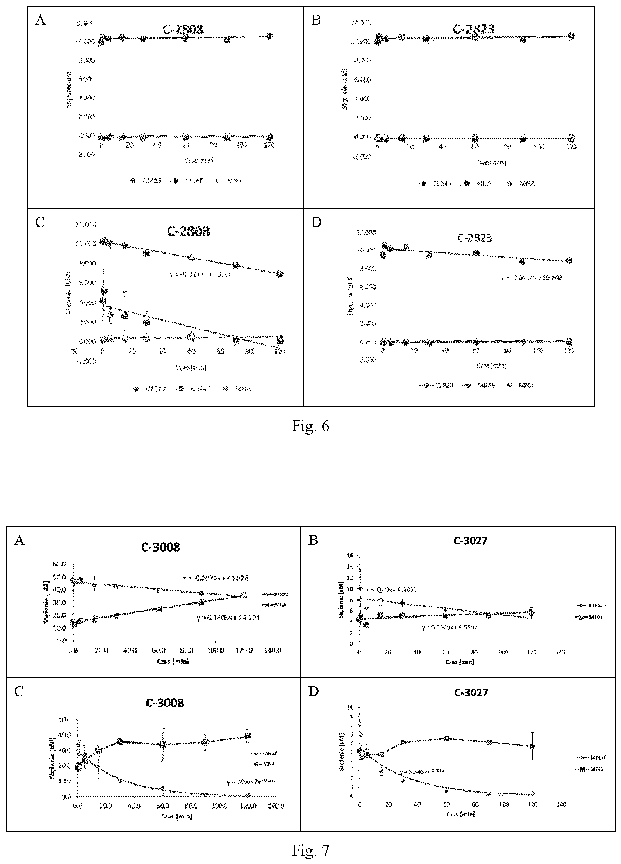

Fig. 2 presents the subgroup of compounds which, according to the invention, constitute analogues- C-2823 and C-2808 - showing chemical stability and resistance to hydrolysis. These compounds in *in vitro* tests showed high stability in PBS buffer. During plasma incubation only slow and slight hydrolysis of these compounds was observed. Fig 6. shows time-concentration dependence curves of studied compounds in PBS and rat plasma. The examined compounds from group 2 show high hydrolytic stability. No changes in concentrations of C-2808 (A) and C-2823 (B) compounds were observed during incubation in PBS solution. During the incubation of compounds in fresh plasma (C and D) there was a slow hydrolysis of the parent compounds. In fig. 6 concentration-time dependence curves of novel compounds hydrolysis are shown - parent compound in blue, intermediate metabolite MNAF in red and the final active metabolite MNA in green.

### (3) Betaine-like compounds releasing MNA

Betaine-like compounds releasing MNA (exemplary structures are shown in fig. 3) similarly to compounds from subgroup (1), under *in vitro* conditions, in plasma immediately decomposed to MNAF, which was then rapidly hydrolysed to MNA. Hydrolysis of MNAF to MNA in plasma involved plasma esterases with the kinetics of the first order reaction. Plasma concentrations of parent substances was below the detection limit, which may indicate total biotransformation of parent compounds to MNAF and MNA. Compounds hydrolyzed to MNAF and MNA also under esterase- free conditions - in PBS, but the decomposition reaction occurred at a lower speed with the kinetics of the zero order elimination (fig.7). Betain group compounds quickly release the intermediate metabolite under *in vitro* conditions, then converted to MNA. In fig. 7 are shown concentration-time curves of the intermediate compound MNAF and MNA after incubation of the sample compounds C-3008 (A) and C-3027 (B) in PBS solution (A) and fresh plasma (C and D) (intermediate metabolite MNAF was marked in blue and MNA in red colour).

It is obvious for a person skilled in pharmacology that compounds being the subject of the invention that release MNA have pharmacological activities as that described for the MNA itself and can be used as drugs, in particular for the treatment or prevention of diseases associated with vascular endothelial dysfunction, defined as an impairment of endothelial nitric oxide (NO) production, which is accompanied by activation of thrombotic and inflammatory processes of the endothelium and blood vessel wall, selected from the group: atherosclerosis in patients with stable coronary artery disease, ischemic brain stroke or limb atherosclerosis, including thromboembolic arteritis; hypercholesterolemia, hypertriglyceridemia; thrombosis other than arterial atherosclerosis, in particular thrombosis associated with implantation of metal vascular prostheses, coronary bypass surgery, haemodialysis, venous thromboembolism; the condition with the current risk factor of atherosclerosis selected from the group: hypercholesterolemia, hypertension, hyperhomocysteinemia, obesity, mental stress, infections, vessel graft atherosclerosis in organ transplantation; chronic heart failure, pulmonary hypertension, nephrotic syndrome, chronic renal failure, adult acute respiratory distress syndrome, cystic fibrosis, chronic obstructive pulmonary disease, pre-eclampsia, erection disorders, Stein-Leventhal syndrome, sleep apnoea, systemic lupus erythematosus, sickle cell anaemia, peptic ulcer disease, glaucoma, chronic liver disease, viral hepatitis, hepatic steatosis, as well as cancer metastasis.

## Claims

1. Pyridine salts derivatives which are O-substituted derivatives of 3-oxymethylaminocarbonyl-1-methylpyridine with formula (I): 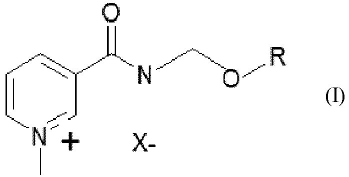 where:
**R** is the substituent selected from the group C(O)R¹ and -(CH₂)R², where:
**R¹** is the substituent selected from a group including: lower straight-chain or branched C1-C4 alkane, adamanthyl, phenyl or trimethoxyphenyl group, lower straight-chain or branched alkane C1-C5 substituted with a COOH or COOR³ group, where:
**R³** is: lower C1-C4 alkane.
**R²** is the substituent selected from the group: H, lower C1-C3 alkane.
**X⁻** is: F⁻, Cl-, Br⁻, I⁻ or other pharmaceutically acceptable counterion.

2. Pyridine salts derivatives according to claim 1 selected from the group:
3-(Acetoxymethylcarbamoyl)-1-methylpyridinium iodide;
3-[(2,2-dimethylpropionyl oxymethyl)carbamoyl]-1-methylpyridinium iodide;
3-(benzoyloxymethylcarbamoyl)-1-methylpyridinium iodide;
3-(isobutanoyloxymethylcarbamoyl)-1-methylpyridinium iodide;
iodide [(1-methylpyridine-1-ium-3-carbonyl)amino]methyl 3,4,5-trimethoxybenzoate;

3. Pyridine salts derivatives according to claim 1 selected from the group:
4- [[(1 -methylpyridin-1 -ium-3 -carbonyl)amino] methoxy]-4-oxobutanoic acid iodide;
3-({[(5-ethoxy-5-oxopentanoyl)oxy]methyl}carbamoyl)-1-methylpyridinium iodide ;
3-({[(4-ethoxy-4-oxobutanoyl)oxy]methyl}carbamoyl)-1-methylpyridinium iodide .

4. Iodide [(1-methylpyridine-1-ium-3-carbonyl)amino]methyl-1-methylcyclohexanecarboxylate.

5. Pyridine salts derivatives according to claims 1 to 4 for use as a medicament.

6. Pyridine salts derivatives according to claims 1 to 4 for use in the treatment or prevention of diseases associated with vascular endothelial dysfunction, defined as an impairment of endothelial nitric oxide (NO) production, which is accompanied by activation of thrombotic and inflammatory processes of the endothelium and blood vessel wall, selected from the group: atherosclerosis in patients with stable coronary artery disease, ischemic brain stroke or limb atherosclerosis, including thromboembolic arteritis; hypercholesterolemia, hypertriglyceridemia; thrombosis other than arterial atherosclerosis, in particular thrombosis associated with implantation of metal vascular prostheses, coronary bypass surgery, haemodialysis, venous thromboembolism; the condition with the current risk factor of atherosclerosis selected from the group: hypercholesterolemia, hypertension, hyperhomocysteinemia, obesity, mental stress, infections, vessel graft atherosclerosis in organ transplantation; chronic heart failure, pulmonary hypertension, nephrotic syndrome, chronic renal failure, adult acute respiratory distress syndrome, cystic fibrosis, chronic obstructive pulmonary disease, pre-eclampsia, erection disorders, Stein-Leventhal syndrome, sleep apnoea, systemic lupus erythematosus, sickle cell anaemia, peptic ulcer disease, glaucoma, chronic liver disease, viral hepatitis, hepatic steatosis, as well as cancer metastasis.

## Patentansprüche

1. Pyridinsalz-Derivate, die O-substituierte 3-Oxymethylaminocarbonyl-1-methylpyridin-Derivate mit der folgenden Formel (I) sind: 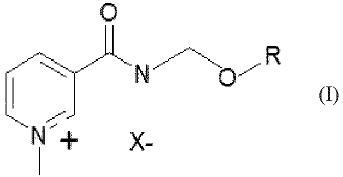 wo:
**R** ein Substituent ausgewählt aus der Gruppe von -C(O)R1 und -(CH2)R2 ist, wobei:
**R¹** ein Substituent ausgewählt aus der Gruppe mit den folgenden Bestandteilen ist: einem niedrigen C1-C4-Alkan mit unverzweigter oder verzweigter Kette, einer Adamantyl-, Phenyl- oder Trimethoxyphenylgruppe, einem niedrigen C1-C5-Alkan mit unverzweigter oder verzweigter Kette, substituiert mit COOH oder COOR³, wobei:
**R³** ein niedriger C1-C4-Alkan ist.
**R²** ein Substituent ausgewählt aus der folgenden Gruppe ist: H, ein niedriges C1-C3-Alkan.
**X⁻** ist: F⁻, Cl-, Br⁻, I⁻ oder ein anderes pharmazeutisch verträgliches Gegenion.

2. Pyridinsalz-Derivate gemäß Anmeldung 1 ausgewählt aus der Gruppe:
3-(Acetoxymethylcarbamoyl)-1-methylpyridiniumiodid;
3 -[(2,2-Dimethylpropionyloxymethyl)carbamoyl]-1-methylpyridiniumjodi;
3-(Benzoyloxymethylcarbamoyl)-1-methylpyridiniumjodid;
3-(Isobutaneoyloxymethylcarbamoyl)-1-methylpyridiniumjodid;
Methyl-3,4,5-trimethoxybenzoat [(1-Methylpyridin-1-um-3-carbonyl)amino] amino] iodid;

3. Pyridinsalz-Derivate gemäß Anmeldung 1 ausgewählt aus der Gruppe:
4-[[(1-Methyl-pyridin-1-um -3 -carbonyl)-amino] -methoxy] -4-oxobutansäurej odid;
3-({[(5-Ethoxy-5-oxopentanoyl)oxy]methyl}carbamoyl)-1-methylpyridiniumiodid;
3-({[(4-Ethoxy-5-oxobutanoyl)oxy]methyl} carbamoyl)-1-methylpyridiniumiodid.

4. [(1 -Metliylpyridin-1-um-3 -carbonyl)amino]methyl-1-methylcyclohexancarboxylatiodid.

5. Pyridinsalz-Derivate gemäß den Anmeldungen 1 bis 4 zur Verwendung als Arzneimittel.

6. Pyridinsalz-Derivate gemäß den Anmeldungen 1 bis 4 zur Vorbeugung von Erkrankungen im Zusammenhang mit vaskulärer endothelialer Dysfunktion, definiert als gestörte Produktion von Stickoxid (NO) durch das Endothel, begleitet von einer Aktivierung thrombo-inflammatorischer Prozesse im Endothel und in der Blutgefäßwand, ausgewählt aus der Gruppe: Atherosklerose bei Patienten mit stabiler koronarer Herzkrankheit, ischämischem Schlaganfall oder Atherosklerose der Extremitäten, einschließlich thromboobliterierender Arteriitis; Hypercholesterinämie, Hypertriglyceridämie; andere Thrombosen als Atherosklerose, insbesondere Thrombose im Zusammenhang mit der Implantation von metallischen Gefäßprothesen, Koronararterien-Bypassoperation, Hämodialyse, venöse Thromboembolie; eine Erkrankung mit einem bestehenden Arteriosklerose-Risikofaktor ausgewählt aus der Gruppe: Hypercholesterinämie, arterielle Hypertonie, Hyperhomocysteinämie, Fettleibigkeit, psychischer Stress, Infektionen, Arteriosklerose bei Organtransplantationen; chronische Herzinsuffizienz, pulmonale Hypertonie, nephrotisches Syndrom, chronisches Nierenversagen, akutes Atemnotsyndrom bei Erwachsenen, Mukoviszidose, chronisch obstruktive Lungenerkrankung, Präeklampsie, erektile Dysfunktion, Stein-Leventhal-Syndrom, Schlafapnoe, systemischer Lupus erythematodes, Sichelzellkrankheit, Magengeschwüre, Glaukom, chronische Lebererkrankungen, Virushepatitis, Fettleber sowie neoplastische Metastasen.

## Revendications

1. Dérivés des sels de pyridine, soit dérivés des O-substitués du 3-oxyméthylaminocarbonyl-1-méthylpyridine ayant la formule suivante (I) : 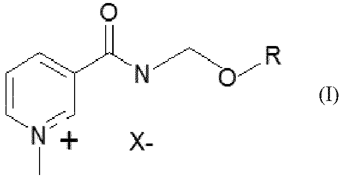 où :
**R** est un substituant sélectionné du groupe C(O)R1 i -(CH2)R2 où :
**R¹** est un substituant sélctionné du groupe comprenant : alcane inférieur C1-C4 linéaire ou ramifié, groupe adamantyle, phényle ou triméthoxyphényle, alcane inférieur C1-C5 linéaire ou ramifié substitué par le groupe COOH ou COOR³ où :
**R³** est un alcane inférieur C1-C4.
**R²** est un substituant sélectionné du groupe : H, alcane inférieur C1-C3.
**X⁻ est un** F⁻, Cl-, Br⁻, I⁻ ou autre contre-ion pharmaceutiquement acceptable.

2. Dérivés des sels de pyridine selon la revendication 1 sélectionnés du groupe :
Iodure de 3-(acétoxyméthylcarbamoyle)-1-méthylpyridine ;
Iodure de 3-[(2,2-diméthylpropionyloxyméthyl)carbamoyle]-1-méthylpyridine ;
Iodure de 3-(benzoyloxyméthylcarbamoyle)-1-méthylpyridine ;
Iodure de 3-(isobutanoyloxyméthylcarbamoyle)-1-méthylpyridine ;
iodure de [(1-méthylpyridine-1-ium-3-carbonyle)amino]méthyle 3,4,5-triméthoxybenzoate ;

3. Dérivés des sels de pyridine selon la revendication 1 sélectionnés du groupe :
Iodure d'acide de 4-[[(1-méthylpyridine-1-ium-3-carbonyle)amino]métoxy]-4-oxybutane ;
Iodure de 3-({[(5-étoxy-5-oxopentanoïque)oxy]méthyl}carbamoyle)-1-méthylpyridine ;
Iodure de 3-({[(4-étoxy-5-oxobutanoïque)oxy]méthyl}carbamoyle)-1-méthylpyridine.

4. Iodure de [(1-méthylpyridine-1-ium-3-carbonyle)amino] méthyl-1-méthylcyklohexancarboxylane.

5. Dérivés des sels de pyridine selon les revendications de 1 à 4 pouvant être utilisés comme médicament.

6. Dérivés des sels de pyridine selon les revendications de 1 à 4 pour le traitement et la préventions des maladies liées à un disfonctionnement de l'endothélium vasculaire définit comme « troubles de la production d'oxyde d'azote (NO) par l'endothélium vasculaire » accompagnées d'une activation des processus thrombotiques et inflammatoires de l'endothélium vasculaire et des parois des vaisseaux sanguins, sélectionnés du groupe : athérosclérose chez les patients souffrant d'une maladie coronarienne stabilisée, accident vasculaire cérébral ou athérosclérose des membres, y compris le thromboangéite oblitérante ; hypercholestérolémie , hypertriglycéridémie ; thrombose autre que l'athérosclérose, notamment la thrombose liée à la mise en place des prothèses vasculaires métalliques, pontage coronarien, hémodialyse, thromboembolie veineuse ; affection avec facteur existant de risque d'athérosclrose sélectionné du groupe : hypercholestérolémie, hypertension artérielle, hyperhomocystéinémie, obésité, stress psychique, infections, athérosclérose liée aux greffes des organes ; insuffisance cardiaque chronique, hypertension pulmonaire, syndrome néphrotique, insuffisance rénale chronique, syndrome de détresse respiratoire aiguë chez les adultes, mucoviscidose, maladie pulmonaire obstructive chronique, prééclampsie, troubles d'érection, syndrome de Stein-Leventhal, apnée du sommeil, lupus érythémateux disséminé, drépanocytose, ulcère, glocaume, maladies chroniques du foie, hépatite, stéatose hépatique aussi bien que métastases cancéreuses.
